# EUROPEAN PATENT APPLICATION

(11) **EP 2 594 635 A1**
(43) Date of publication of application: **22.05.2013**
(21) Application number: 11306515.5
(22) Date of filing: 18.11.2011
(51) Int. Cl.: C12N 5/077, G01N 33/50

(54) **Method for generating primate cardiovascular progenitor cells for clinical and drug cells testing use from primate embryonic stem cells or embryonic-like state cells, and their applications**

(71) Applicant: Univercell Biosolutions, 31400 Toulouse (FR)
(72) Inventor: Costecalde, Guillaume, 31460 Caraman (FR); Fontaine, Vincent, 91270 Vigneux-Sur-Seine (FR)
(74) Representative: Regimbeau

(57) **Abstract**

The present invention is directed to a method for the *in vitro* preparation of cardiovascular progenitors cells from mammalian embryonic stem cells (ES cells) or mammalian embryonic-like state cells, preferably from human, wherein said method comprises the use of reconstituted basement membrane-derived extracellular composition (named "matrigel"), particularly from EHS (Engelbreth Holm-Swarm) tumor extract and the SSEA1 marker as a positive cardiovascular progenitors differentiation marker with BMP-2. Derived cell culture medium compositions and kit in relation with the method of the invention or product obtainable by said method form also part of the present invention. The present invention is further directed to method for testing agents for effect on human cardiovascular cells.

SEQUENCE LISTING

<110>  UNIVERCELL BIOSOLUTIONS

<120>  Method for generating primate cardiovascular progenitor cells for
        clinical and drug cells testing use from primate embryonic stem
        cells or embryonic-like state cells, and their applications

<130>  69572

<160>  42

<170>  PatentIn version 3.5

<210>  1
<211>  25
<212>  DNA
<213>  artificial sequence

<220>
<223>  PCR forward primer used for ß-tubulin marker

<400>  1
ccggacagtg tggcaaccag atcgg                                          25


<210>  2
<211>  25
<212>  DNA
<213>  artificial sequence

<220>
<223>  PCR reverse primer used for ß-tubulin marker

<400>  2
tggccaaaag gacctgagcg aacgg                                          25


<210>  3
<211>  20
<212>  DNA
<213>  artificial sequence

<220>
<223>  PCR forward primer used for Nkx2.5 marker

<400>  3
catttacccg ggagcctacg                                                20


<210>  4
<211>  25
<212>  DNA
<213>  artificial sequence

<220>
<223>  PCR reverse primer used for Nkx2.5 marker

<400>  4
gctttccgtc gccgccgtgc gcgtg                                          25


<210>  5
<211>  25
<212>  DNA

<213>  artificial sequence

<220>
<223>  PCR forward primer used for Mef2c marker

<400>  5
agatacccac aacacaccac gcgcc                                          25


<210>  6
<211>  21
<212>  DNA
<213>  artificial sequence

<220>
<223>  PCR reverse primer used for Mef2c marker

<400>  6
atccttcaga gagtcgcatg c                                              21


<210>  7
<211>  28
<212>  DNA
<213>  Artificial Sequence

<220>
<223>  PCR forward primer used for SRF marker

<400>  7
ctccgccccg ctcagacccc accacaga                                       28


<210>  8
<211>  23
<212>  DNA
<213>  artificial sequence

<220>
<223>  PCR reverse primer used for SRF marker

<400>  8
aggtagttgg tgatggggaa gga                                            23


<210>  9
<211>  24
<212>  DNA
<213>  artificial sequence

<220>
<223>  PCR forward primer used for a-actin marker

<400>  9
ctatgtcgcc ctggattttg agaa                                           24


<210>  10
<211>  23
<212>  DNA
<213>  artificial sequence

<220>
<223>  PCR reverse primer used for a-actin marker

<400>  10
tgagggaagg tggtttggaa gac                                              23


<210>  11
<211>  21
<212>  DNA
<213>  artificial sequence

<220>
<223>  PCR forward primer used for Oct-4 marker

<400>  11
acgaccatct gccgctttga g                                                21


<210>  12
<211>  20
<212>  DNA
<213>  artificial sequence

<220>
<223>  PCR reverse primer used for Oct-4 marker

<400>  12
gcctctcact cggttctgat                                                  20


<210>  13
<211>  24
<212>  DNA
<213>  artificial sequence

<220>
<223>  PCR forward primer used for Tbx6 marker

<400>  13
aggcccgcta cttgtttctt ctgg                                             24


<210>  14
<211>  24
<212>  DNA
<213>  artificial sequence

<220>
<223>  PCR reverse primer used for Tbx6 marker

<400>  14
tggctgcata gttgggtggc tctc                                             24


<210>  15
<211>  23
<212>  DNA
<213>  artificial sequence

<220>
<223>  PCR forward primer used for Isl1 marker

<400>  15
catcgagtgt ttccgctgtg tag                                              23


<210>  16

<211>  24
<212>  DNA
<213>  artificial sequence

<220>
<223>  PCR reverse primer used for Isl1 marker

<400>  16
gtggtcttct ccggctgctt gtgg                                              24


<210>  17
<211>  20
<212>  DNA
<213>  artificial sequence

<220>
<223>  PCR forward primer used for FoxH1 marker

<400>  17
gcccctgccc acgctgtcta                                                   20


<210>  18
<211>  22
<212>  DNA
<213>  artificial sequence

<220>
<223>  PCR reverse primer used for FoxH1 marker

<400>  18
ggtacctctt cttcctcctc tt                                                22


<210>  19
<211>  22
<212>  DNA
<213>  artificial sequence

<220>
<223>  PCR forward primer used for Brachyury marker

<400>  19
cggaacaatt ctccaaccta tt                                                22


<210>  20
<211>  22
<212>  DNA
<213>  artificial sequence

<220>
<223>  PCR reverse primer used for Brachyury marker

<400>  20
gtactggctg tccacgatgt ct                                                22


<210>  21
<211>  19
<212>  DNA
<213>  artificial sequence

<220>

<223>  PCR forward primer used for Mesp1 marker

<400>  21
ctcgtctcgt ccccagact                                                    19


<210>  22
<211>  19
<212>  DNA
<213>  artificial sequence

<220>
<223>  PCR reverse primer used for Mesp1 marker

<400>  22
agcgtgcgca tgcgcagtt                                                    19


<210>  23
<211>  22
<212>  DNA
<213>  artificial sequence

<220>
<223>  PCR forward primer used for Tbx20 marker

<400>  23
ctgagccact gatccccacc ac                                                22


<210>  24
<211>  22
<212>  DNA
<213>  artificial sequence

<220>
<223>  PCR reverse primer used for Tbx20 marker

<400>  24
ctcaggatcc acccccgaaa ag                                                22


<210>  25
<211>  25
<212>  DNA
<213>  artificial sequence

<220>
<223>  PCR forward primer used for Gata4 marker

<400>  25
ggttcccagg cctcttgcaa tgcgg                                             25


<210>  26
<211>  25
<212>  DNA
<213>  artificial sequence

<220>
<223>  PCR reverse primer used for Gata4 marker

<400>  26
agtggcattg ctggagttac cgctg                                             25

<210>  27
<211>  20
<212>  DNA
<213>  artificial sequence

<220>
<223>  PCR forward primer used for Pax6 marker

<400>  27
gccagcaaca cacctagtca                                                    20


<210>  28
<211>  20
<212>  DNA
<213>  artificial sequence

<220>
<223>  PCR reverse primer used for Pax6 marker

<400>  28
tgtgagggct gtgtctgttc                                                    20


<210>  29
<211>  28
<212>  DNA
<213>  artificial sequence

<220>
<223>  PCR forward primer used for a-FP marker

<400>  29
actgcaattg agaaacccac tggagatg                                           28


<210>  30
<211>  25
<212>  DNA
<213>  artificial sequence

<220>
<223>  PCR reverse primer used for a-FP marker

<400>  30
cgatgctgga gtgggctttt tgtgt                                              25


<210>  31
<211>  22
<212>  DNA
<213>  artificial sequence

<220>
<223>  PCR forward primer used for Cx43 marker

<400>  31
taccatgcga ccagtggtgc gc                                                 22


<210>  32
<211>  23
<212>  DNA
<213>  artificial sequence

<220>
<223>  PCR reverse primer used for Cx43 marker

<400>  32
gaattctggt tatcatcggg gaa                                              23


<210>  33
<211>  22
<212>  DNA
<213>  artificial sequence

<220>
<223>  PCR forward primer used for GAPDH marker

<400>  33
atgggcaagg tgaaggtcgg ag                                               22


<210>  34
<211>  22
<212>  DNA
<213>  artificial sequence

<220>
<223>  PCR reverse primer used for GAPDH marker

<400>  34
tcgcccgact tgattttgca gg                                               22


<210>  35
<211>  19
<212>  DNA
<213>  artificial sequence

<220>
<223>  PCR forward primer used for Tbx5 marker

<400>  35
taccaccaca cccatcaac                                                   19


<210>  36
<211>  20
<212>  DNA
<213>  artificial sequence

<220>
<223>  PCR reverse primer used for Tbx5 marker

<400>  36
acaccaagac agggacagac                                                  20


<210>  37
<211>  22
<212>  DNA
<213>  artificial sequence

<220>
<223>  PCR forward primer used for LeftyA marker

<400>  37

gggaattggg atacctggat tc                                                22

<210>  38
<211>  22
<212>  DNA
<213>  artificial sequence

<220>
<223>  PCR reverse primer used for LeftyA marker

<400>  38
taaatatgca cgggcaaggc tc                                                22

<210>  39
<211>  20
<212>  DNA
<213>  artificial sequence

<220>
<223>  PCR forward primer used for TDGF1 marker

<400>  39
acagaacctg ctgcctgaat                                                   20

<210>  40
<211>  20
<212>  DNA
<213>  artificial sequence

<220>
<223>  PCR reverse primer used for TDGF1 marker

<400>  40
atcacagccg ggtagaaatg                                                   20

<210>  41
<211>  20
<212>  DNA
<213>  artificial sequence

<220>
<223>  PCR forward primer used for Nanog marker

<400>  41
caaaggcaaa caacccactt                                                   20

<210>  42
<211>  20
<212>  DNA
<213>  artificial sequence

<220>
<223>  PCR reverse primer used for Nanog marker

<400>  42
tctgctggag gctgaggtat                                                   20

## Description

The present invention is directed to a method for the *in vitro* preparation of cardiovascular progenitors cells from mammalian embryonic stem cells (ES cells) or mammalian embryonic-like state cells, preferably from human, wherein said method comprises the use of reconstituted basement membrane-derived extracellular composition (named "matrigel"), particularly from EHS (Engelbreth Holm-Swarm) tumor extract and the CD15 (SSEA1) marker as a positive cardiovascular progenitors differentiation marker. Derived cell culture medium compositions in relation with the method of the invention or product obtainable by said method form also part of the present invention. The present invention is directed to method for testing agents for effect on human cardiovascular cells and for clinical applications.

Heart failure is becoming a predominant disease and a leading cause of death in most of developed countries. Regardless of the origin of myocardial failure (i.e, ischemic or genetic), the clinical symptoms result mainly from the death of cardiomyocytes replaced by a fibrotic and non contractile tissue. Stem cell source to achieve myocardial regeneration have been the object of great interest for the last decade. Among various stem cell types, embryonic stem (ES) cells (Behfar A et al., FASEB J., 2002, 16:1558-1566; Ménard C et al., The Lancet, 2005, 17-23; 366(9490): 1005-12; and Singla DK et al., J Mol Cell Cardiol., 2006, 40:195-200) or ES cell-derived cardiomyocytes (Laflamme MA et al., Am J Pathol., 2005, 167:663-71; and Kolossov E et al., J Exp Med., 2006, 203:2315-27) have turned out to be the most promising for replacing scar fibrosis by new contractile elements.

Number of cells are required to regenerate a post-infarcted human myocardium (i.e., several hundreds of million). It has been shown recently that proliferative mouse ES cells engrafted in a diseased myocardium further differentiate into functional cardiomyocytes following *in-vitro* commitment using the cardiogenic morphogen BMP-2 (Behfar A et al, 2002; Ménard C et al., 2005; and Singla DK et al., 2006). Cardiac-specified cells then complete their differentiation in response to the local cues present in the scar and do not generate any kind of tumors.

More imminent in term of application, however, is the employment of stem cell technologies for drug discovery and development. Novel improved *in vitro* cell models based on physiologically relevant human cells will result in better precision and more cost-effective assays ultimately leading to lower attrition rates and safe new drugs. A prevailing issue in drug discovery is the attrition of new chemical candidates due to ADME, pharmakocinetics and safety concerns, often in advance of mechanistic evaluation. From 1991 to 2000 an attrition rate of 30% was reported for compounds because of toxicology and clinical safety. A significant portion of this attrition is due to cardiotoxicity (Schuster, D. and al. (2005), Curr. Pharm. Des. 11, 3545-3559). Mouse ES cells can be fairly routinely differentiated into beating cardiomyocytes but demonstrate their limits to drastically reduce the attrition rate. Although progress has been made, human stem cells that reproducibly differentiates into cardiomyocytes with predictive pharmacology readouts for safety screens are very limited. There is growing interest in human stem cells to deliver this important resource that should result in an earlier identification of liable compounds pre-clinically. This trend is also largely accelerated by the urgent need of finding alternative methods and reducing animal use for toxicity testing.

More recently, Leschik et al (Nature protocols,3, 9, 1381-1387, 2008) (see also the patent document WO2009/112496 have demonstrated that primate ES cells such as Human ES cells or embryonic-like state cells, can also be directed toward a cardiogenic and vascular fate using the morphogen BMP-2 in association with a receptor tyrosine kinase inhibitor, particularly the SU5402 or SU112248 compound, and to selectively collect these cardiovascular progenitors thus obtained by using the positive SSEA1 biomarker.

The SU5402 compound has the following formula: 3-[3-(2-Carboxyethyl)-4-methylpyrrol-2-methylidenyl]-2-indolinone, and the SU11248 compound (also referenced as "Sunitinib malate" or Sutent" has the following formula: N-[2-(diethylamino)ethyl]-5-[(Z)-(5-fluoro-1,2-dihydro-2-oxo-3H-indol-3-ylidine)methyl]-2,4-dimethyl-1H-pyrrole-3-carboxamide.

When sorted, these cardiovascular progenitors express a series of genes (T, Tbx5, Tbx6, Mesp, Is11, Tbx20, Nkx2.5, Mef2c, Myocardin, Tbx18) and proteins (Tbx6, Mesp1/2). After several days in culture, they further express some markers (Isl-1, Nkx2.5, Mef2C and Tbx5 but not the smooth muscle actin marker α-SMA). These cardiovascular progenitors differentiate into cardiac cells (Cx43, MLC2v, α-actinin, cardiac troponine T and β-MHC markers) by the use of a growth factor mixture with a certain timing. Moreover, these cardiovascular progenitors can also differentiate into smooth muscle cells (α-SMA marker) and endothelial cells (CD31 marker) under certain conditions (VEGF and PDGF treatments respectively) providing new source of cells for neovascularisation tests or revascularisation treatment after ischemia and valuable tool for compounds testing.

Primate ES cells or embryonic-like state cells lines require feeder cells, such as Mouse Embryonic Fibroblasts (MEFs) and/or FGF to maintain their pluripotency and to allow their proliferation before the step of their differentiation. Because of FGFs (particularly FGF-2) and TGFβ production by these feeder cells, or their addition in the medium culture, the use of an RTK inhibitor such as SU5402 or SU112248 is necessary during BMP-2 treatment in the differentiation step.

Thus, there is a need, to provide a new method for the preparation of such cardiovascular progenitors which can subsequently differentiate into cardiomyocytes, smooth muscles cells and endothelial cells without any sign of hyperproliferation and without implementing RTK inhibitor.

This is the object of the present invention.

The inventors have demonstrated that the use of reconstituted basement membrane-derived extracellular composition, particularly from EHS (Engelbreth Holm-Swarm) tumor extract in association with BMP-2 for the treatment of primate stem cells or iPS (induced Pluripotent Stem cells) cells during the step of generation of cardiovascular progenitors allows said generation without the need to add RTK inhibitor in the culture medium.

Particularly, the use of EHS tumor extract "matrigel" (BD Matrigel™ Basement Membrane Matrix) allows to remove RTK inhibitors without any difference in BMP-2 treatment efficiency (percentage of SSEA1 positive cells) compared to MEFs.

These data open the path for the use of early cardiovascular progenitors prepared without the need of RTK inhibitors addition, which retain the capability to proliferate and repopulate the postinfarction scar and subsequently differentiate into cardiomyocytes, smooth muscles cells and endothelial cells which also retain the capacity to proliferate and repopulate the post infarction scar and is a valuable resource to screen new drug to test their predictive toxicity or efficacy of the clinical situation on the 3 differentiated cells.

Thus, the present invention relates to new methods for obtaining substantially pure populations of primate cardiovascular progenitors as well as compositions such as culture medium compositions or therapeutic compositions containing these cell populations and method of using these cell populations.

In the present description, it will be designated by "cardiac progenitors cells" or "cardiovascular progenitors cells", words which have the same meaning and that will be used interchangeably here, cells capable of generating cardiomyocytes, smooth muscle cells and endothelial cells. These cardiovascular progenitors cells can differentiate into the three cardiovascular lineages: cardiac (Cx43, MLC2v, α-actinin, cardiac troponine T and β-MHC markers), endothelial cells (CD31+ marker) and smooth muscle cells (α-SMA marker).

B y "cardiomyocytes generated from cardiovascular progenitors" (or "cardiomyocytes derived thereof"), it can be particularly designated later-stage cardiomyocytes precursors (than the cardiovascular progenitor cells obtained by the method of the present invention) or terminally cardiomyocyte differentiated cells obtained from said cardiovascular progenitors.

By "smooth muscle cells generated from cardiovascular progenitors" (or "smooth muscle cells derived thereof"), it can be particularly designated later-stage smooth muscle cells precursors (than the cardiovascular progenitor cells obtained by the method of the present invention) or terminally smooth muscle cells differentiated cells obtained from said cardiovascular progenitors.

By "endothelial cells generated from cardiovascular progenitors" (or "endothelial cells derived thereof"), it can be particularly designated later-stage endothelial cells precursors (than the cardiovascular progenitor cells obtained by the method of the present invention) or terminally endothelial cells differentiated cells obtained from said cardiovascular progenitors.

By "pluripotency", it is intended to designate herein pluripotent ES cells-derived cells that are the descendants of totipotent embryonic stem cells and can differentiate into cells derived from any of the three germ layers ectoderm, endoderm and mesoderm.

By iPS cells, it is intended to designate pluripotent stem cell artificially derived from a non-pluripotent cell, typically an adult somatic cell, by inducing expression of certain genes.

By iPS cells, it is intended also to designate somatic cells, such as fibroblast cells capable of being, or being, transformed in the desired cardiomyocyte phenotype by transcriptome transfer (named tcardiomyocytes, see Kim et al., PNAS direct submission, accession n°.GSE30164, (www.pnas.org/cgi/doi/10.1073/pnas. 1101223108).

By "Feeder cells" it is intended to designate cells that are co-cultured with the primate ES cells. They provide an environment in which the primate ES cells type can grow. The culture of the primate ES cells can be supported by primary mouse embryonic fibroblasts (MEF) or primary human foreskin fibroblasts as exemplified. Immortalized mouse embryonic fibroblasts or human foreskin fibroblasts cells can also be used as feeder cells.

In a first aspect, the invention is directed to an *in vitro* method for the preparation of cardiovascular progenitor cells from mammalian ES cells or from mammalian embryonic-like state cells, preferably for the preparation of a substantially purified population of cardiovascular progenitors, wherein said method comprises the following step of:
a) culturing of mammalian ES cells or embryonic-like state cells in a medium containing suitable agents allowing their proliferation and maintaining their pluripotency;
b) differentiating the mammalian pluripotent ES cells or embryonic-like state cells obtained in step a) toward cardiovascular progenitors cells by suspending said pluripotent ES cells or embryonic-like state cells in a medium containing BMP-2 (Bone Morphogenetic Protein 2);
c) selecting and collecting the differentiated mammalian ES cells or mammalian embryonic-like state cells obtained in step b) which display the SSEA1 marker at their membrane surface, the mammalian ES cells or embryonic-like state cells displaying said SSEA1 marker being selecting and collecting as cardiovascular progenitors cells, wherein in step b) said medium containing BMP-2 further contains a reconstituted basement membrane-derived extracellular composition from EHS tumor extract (named "matrigel" in the present application).

In a preferred embodiment, this matrigel in step b) is a reconstituted basement membrane-derived extracellular composition from EHS tumor extract.

In a more preferred embodiment, this matrigel in step b) is a reconstituted basement membrane-derived extracellular composition containing in parts by weight about 60-85% laminin, 5-30 % collagen IV, 1-10 % nidogen, 1-10 % heparan sulfate proteoglycan and 1-5 % entactin and wherein said composition can polymerize into a rigid stable gel in at least 20 minutes on heating at 24 °C-32 °C.

In a more preferred embodiment, this matrigel in step b) is a reconstituted basement membrane-derived extracellular composition from EHS tumor extract, more preferably obtained by a process carried out at about 4 °C and comprising the steps of:
(a) homogenizing the tumor multiple times in a suitable first buffer containing at least 3.4 M salt and discarding the soluble fraction after each homogenization so as to remove undesirable components;
(b) extracting the residual tumor in a suitable urea buffer containing at least 2M urea keeping the extract stirred for about 16-18 hours;
(c) separating the extract form step (b) and saving the extract while repeating step (b) with the residual tumor fraction and again saving the extract resulting therefrom;
(d) combining the extracts from step (c) and dialyzing against a suitable sterilizing buffer with multiple changes of the dialyzing buffer;
(e) further dialyzing against a suitable buffer not containing a sterilizing component;
(f) recovering a dialysate from step (e); and
(g) polymerizing the dialysate at 24 °C-32 °C for at least 20 minutes.

In an also more preferred embodiment, this matrigel in step b) is a reconstituted basement membrane-derived extracellular composition from EHS tumor extract, wherein said EHS tumor extract is obtained from an extract of EHS cell culture of EHS cells from the EHS strain selected from the group consisted of strain line ATCC number 63020 (including an insert coding for collagen type IV, alpha 1 [Col4a1]), ATCC number 63022 (including an insert coding for vimentine), ATCC number 63128 (including an insert coding for nidogene).

In a preferred embodiment, said matrigel is the BD Matrigel™ Basement Membrane Matrix from BD Biosciences, (such as the matrigel contained in the reference BD Biosciences cat n°356231).

In a preferred embodiment, said embryonic-like state cells are induced pluripotent stem cells, commonly abbreviated as iPS cells, preferably from adult somatic cells, particularly from adult or foetal fibroblasts (i.e. pulmonary foetal fibroblasts strain IMR90, ref. ATCC No. CCL-186), from keratinocytes, from astrocytes or from peripheral blood cells.

In a more preferred embodiment, said iPS cells are obtained using human dermal fibroblasts infected by lentivirus harbouring the cDNAs encoding Oct4, Sox2, Lin 28, Klf4 and Nanog, preferably under ES cells culture conditions (Maherali N et al., 2008, Stem Cell 3 345-350).

The preparation of iPS cells from mammalian cells, particularly from mouse cells or from human cells is well known from the skilled person (Takahashi K, et al., Cell. 2007 Nov 30; 131(5):861-72; Nakagawa et al., Nat Biotechnol. 2008; 26(1):101-6); Wemig et al., Nature, 2007, 448(7151):318-24; and Maherali N et al., 2008).

In a preferred embodiment, the invention is directed to an *in vitro* method according to the present invention wherein said mammalian cells are primate, mouse or rat cells, preferably primate cells and more preferably human cells.

Primate embryonic stem cells can be isolated from blastocysts of members of the primate species (see for example U.S. Pat. No. 5,843,780; Thomson et al., Proc. Natl. Acad. Sci. USA 92:7844, 1995). Human embryonic stem (hES) cells can be prepared from human blastocyst cells using the techniques described by Thomson et al. (U.S. Pat. No. 6,200,806; Science 282:1145, 1998; Curr. Top. Dev. Biol. 38:133 ff., 1998) and Reubinoff et al., Nature Biotech., 18:399, 2000).

Cell culture methods are described generally in the current edition of Culture of Animal Cells: A Manual of Basic Technique (R. I. Freshney ed., Wiley & Sons); General Techniques of Cell Culture (M. A. Harrison & I. F. Rae, Cambridge Univ. Press), and Embryonic Stem Cells: Methods and Protocols (K. Turksen ed., Humana Press). Tissue culture supplies and reagents are available from commercial vendors such as Gibco/BRL, Nalgene-Nunc International, Sigma Chemical Co., and ICN Biomedicals.

In a preferred embodiment, said BMP-2 protein (also named BMP2) is the human BMP-2, more preferably a recombinant hBMP-2, more preferably the human BMP-2 protein having the amino acids sequence depicted in GenBank Accession Number AAF21646.

In a preferred embodiment, the CD-15 marker is the human CD-15 (also named "SSEA1" marker, "SSEA1" for "Stage Specific Embryonic Antigen 1").

In a preferred embodiment the invention provides a method for preparing a substantially pure population of progenitor cells which is at least about 80 %, preferably 85 %, 90 %, 95 %, 97 %, 98 % and 99 % pure cardiovascular progenitor cells which display at their surface the marker SSEA1 and, preferably, which retain their capability to proliferate and repopulate the postinfarction scar after administration to a patient.

In step a) of the method according to the invention, medium for culturing primate ES cells or primate embryonic-like state cells allowing their proliferation and maintaining their pluripotenty are well known by the skilled person (see cell culture methods described in the above referenced manuals and publications).

For example, it is known that primate ES cells required feeder cells for their culture, such as mouse embryonic fibroblasts, and FGF2 (bFGF) to maintain their pluripotency.

Medium allowing feeder free cells condition of ES or iPS cell culture are selected, but not limited to, from the following group of Nutristem™ medium, Stempro (Invitrogen), ReproFF2 (Reprocell), StemMedia™ Nutristem™ XF/FF (Stemgent), mTeSR^{®}1, TeSR™2 (Stem cells technologies), AF Nutritem^{®} hESC XF (Biological industries). Standard media allowing feeder free cells condition of culture are well known from the skilled person

For example of basic medium which can be used for culturing primate ES cells or primate embryonic-like state cells, the KO™-DMEM medium from Invitrogen (or from Gibco, Rockville, MD, USA) can be cited, Nutristem™ can also be used.

It is preferred that FGF2 (fibroblast growth factor 2, also named bFGF) is added to the basic medium after a period of at least an overnight with ES cell or embryonic-like state cells basic medium without FGF2 on feeder cell layer.

It is preferred that the culture medium is changed every day.

Generally, the cell colonies are dissociated into single cells or small cell clusters every 4-5 days using trypsin or collagenase depending on the primate ES cells or primate embryonic-like state cells source (monkey or human source).

It is preferred to never let the ES cells or primate embryonic-like state cells colonies reaching confluency in the dish.

In step a) of the method according to the invention, the medium for culturing primate ES or primate embryonic-like state cells comprised a basic medium, such as the KO™-DMEM medium, which can be supplemented with platelet lysate obtained from primate blood, preferably from human blood.

In step b) of the method according to the invention, the basic medium for differentiating primate ES cells or primate embryonic-like state cells can be selected for example in the group consisting of RPMI or DMEM medium.

In a preferred embodiment, the primate stem cells or embryonic-like state cells, can be treated for respectively 96 H and 6 days with 10 ng/ml BMP-2 in RPMI supplemented with 2 % B27, 0.5 % human albumin and on matrigel as described above.

It is preferred that the cells do not reach confluency at the end of BMP-2 treatment. It is also preferred that BMP-2 is added as soon as small colonies of ES cells appear.

Preferably, in step c), the anti-SSEA1 antibodies are anti-human SSEA1 antibodies, most preferably, monoclonal antibodies.

In a preferred embodiment, the anti-SSEA1 antibodies are labelled, more preferably with a marker which can be used to select and to separate the cells displaying the SSEA1 marker from a cells population, preferably from a cells population obtained or susceptible to be obtained by the steps a) and b) of the method for the preparation of a population of cardiovascular progenitors cells from primate ES cells or embryonic-like state cells according to the present invention.

More preferably, said antibody marker is a fluorescent marker such as FITC.

In another preferred embodiment, the anti-SSEA1 antibodies are bound at the surface of magnetic beads or articles or coupled to magnetic compounds.

Method using magnetic beads or particles is usually implemented for sorting cells. Antibodies specific for a particular cell of interest are covalently bond to magnetic particles. They then incubate a mixture of cells in a solution with the magnetic antibodies. The entire reaction mixture is exposed to a magnetic field, which retains the cells of interest. Some of these particles are even composed of materials that naturally degrade without adversely affecting cell function. Such a system to separate or enrich population in specific cell is well known from the skilled man (see BD Biosciences system, (San Jose, CA USA); positive cells selection with the MidiMACS™ separation system from Miltenyi Biotech (Bergisch Gladbach, Germany), Polysciences or Dynal Biotech)).

As alternatives in cell separation, the well established fluorescence-activated cells sorting (FACS) technique can be also used (Flow cytometers can measure and separate up to 500,000 cells per minute).

The invention also includes a substantially purified population of cardiovascular progenitor cells susceptible to be obtained by the method of the present invention.

In certain embodiment, the cells of the cardiovascular progenitor cells population according to the present invention express the early mesodermal Brachyury, Mesp-1 and Tbx6 markers, the cardiac Tbx20 and Mef2c markers, the GATA4, Nkx2.5, Isl1, Tbx5, Tbx18, MyoCD, Flk-1 markers and the Oct-4A marker.

These cells can differentiate into the three cardiovascular lineages: cardiac (Cx43, MLC2v, α-actinin, cardiac troponine T and β-MHC markers), endothelial cells (CD31+ marker) and smooth muscle cells (α-SMA marker).

Preferably, by substantially purified population is meant that greater than about 80 % of the cells are cardiovascular progenitor cells, preferably greater than about 90 %, more preferably greater than about 95 %, more preferably yet greater than about 98 % and most preferably greater than about 99 %.

The substantially purified population of cardiovascular progenitor cells of this invention is useful for many clinical applications, preferably as a therapeutical composition or a medicament.

In a preferred embodiment, the substantially purified population of cardiovascular progenitor cells according to the present invention is a population of human cardiovascular progenitor cells obtained by the method of the present invention, wherein said cardiovascular progenitor cells are progenitors of cardiomyocytes, endothelial cells, and vascular smooth muscle cells.

More preferably, said population of human cardiovascular progenitor cells of the present invention is obtained by culturing ES cells or embryonic like state cells in serum free media in the presence of BMP-2 and a reconstituted basement membrane derived extracellular composition from a tumor, preferably from EHS tumor extract and obtained by a process carried out at about 4 °C (4 °C ± 2 °C).

Is also preferred a composition comprising a population of human cardiovascular precursors according to the invention.

The invention particularly concerns the use of the purified population of primate cardiovascular progenitor cells of this invention, preferably a population of human cells, for preparing a therapeutic composition for replacing or regenerating cells or tissues in a primate, particularly cardiac, smooth muscles and endothelial cells. In particular, the invention concerns the use of a population of human cardiovascular progenitor cells of this invention differentiated from mammalian ES cells or from mammalian embryonic-like state cells, such as human embryonic stem cells or human iPS cells, for treating heart failure in a human.

In a particular embodiment, the present invention comprises a composition comprising the purified population of primate cardiovascular progenitor cells obtained by the method of the present invention for its use to enhance tissue maintenance or repair of cardiac muscle for any perceived need, such as an inborn error in metabolic function, the effect of a disease condition, or the result of significant trauma.

The present invention also comprises a method of cardiomyocytes replacement comprising administering to a subject in need of such replacement a composition comprising cardiomyocytes, or cardiomyocytes derived from cardiovascular progenitor cells, produced by the method of the present invention

The present invention further comprises a method of treating a disorder characterized by insufficient cardiac function comprising administering to a subject in need of such treatment a composition comprising human cardiovascular progenitor cells according to the present invention.

In order to determine the suitability of cell compositions for therapeutic administration, the cells can first be tested in a suitable animal model. At one level, cells are assessed for their ability to survive and maintain their phenotype *in vivo.* Cell compositions are administered to immunodeficient animals (such as nude mice, or animals rendered immunodeficient chemically or by irradiation). Tissues are harvested after a period of regrowth, and assessed as to whether said cardiovascular progenitor derived cells are still present.

This can be performed by administering cells that express a detectable label (such as green fluorescent protein, or β-galactosidase); that have been prelabeled (for example, with BrdU or [3H]thymidine), or by subsequent detection of a constitutive cell marker (for example, using human-specific antibody). The presence and phenotype of the administered cells can be assessed by immunohistochemistry or ELISA using human-specific antibody, or by RT-PCR analysis using primers and hybridization conditions that cause amplification to be specific for human polynucleotides, according to published sequence data.

The suitability can also be determined by assessing the degree of cardiac recuperation that ensues from treatment with a cell population of said cardiovascular progenitor derived cardiomyocytes. A number of animal models are available for such testing and well known from the skilled person.

Said composition comprising the purified population of primate cardiovascular progenitor cells obtained by the method of the present invention can be used for tissue reconstitution or regeneration in a human patient or other subj ect in need of such treatment. The cells are administered in a manner that permits them to graft or migrate to the intended tissue site and reconstitute or regenerate the functionally deficient area.

In a preferred embodiment, said cells can be administered to reconstitute or regenerate muscle such as heart muscle or femoral muscle (differentiation into the smooth muscle cells cardiovascular lineage)

In another preferred embodiment, said cells are administered to organ revascularization, such as heart (differentiation into the endothelial cells cardiovascular lineage)

Special devices are available that are adapted for administering cells capable of reconstituting cardiac function directly to the chambers of the heart, the pericardium, or the interior of the cardiac muscle at the desired location.

Medical indications for such treatment include treatment of acute and chronic heart conditions of various kinds, such as coronary heart disease, cardiomyopathy, endocarditis, congenital cardiovascular defects, heart valves disease and congestive heart failure. Efficacy of treatment can be monitored by clinically accepted criteria, such as reduction in area occupied by scar tissue or revascularization of scar tissue, and in the frequency and severity of angina; or an improvement in developed pressure, systolic pressure, end diastolic pressure, Δpressure/Δtime, patient mobility, and quality of life.

Said composition comprising the purified population of primate cardiovascular progenitor cells obtained by the method of the present invention can be supplied in the form of a pharmaceutical composition, comprising a pharmaceutical acceptable excipient. Choice of the cellular composition excipient and any accompanying elements of the composition will be adapted in accordance with the route and device used for administration. The composition may also comprise or be accompanied with one or more other ingredients that facilitate the engraftment or functional mobilization of the cardiomyocytes generated from said cardiovascular progenitor cells obtained by the method of the present invention.

Suitable ingredients include matrix proteins that support or promote adhesion of said generated cardiomyocytes, or precursors thereof.

In another aspect, the present invention is directed to the use of the purified population of primate, preferably human, cardiovascular progenitor cells obtained by the method of the present invention, or generated cardiomyocytes, smooth muscles or endothelial cells derived thereof, for identifying compounds having potential or no potential specific effects on cardiomyocytes, smooth muscles, or endothelial cells particularly toxic effect or pharmacological effect on cell function.

Said population of primate, preferably human, cardiovascular progenitor cells obtained by the method of the present invention, or cardiomyocytes, smooth muscles or endothelial cells derived thereof, can be used to screen for factors (such as solvents, small molecule drugs, peptides, oligonucleotides) or environmental conditions (such as culture conditions or manipulation) that affect the characteristics of such cells and their various progeny.

Said population of primate, preferably human, cardiovascular progenitor cells obtained by the method of the present invention, or cardiomyocytes, smooth muscles or endothelial cells derived thereof, can be used to screen factors that promote maturation into later-stage cardiomyocytes, smooth muscles or endothelial cells precursors, or terminally differentiated cells, or to promote proliferation and maintenance of such cells in long-term culture. For example, candidate maturation factors or growth factors are tested by adding them to cells in different wells, and then determining any phenotypic change that results, according to desirable criteria for further culture and use of the cells.

Form also part of the present invention, other screening applications of the population of primate, preferably human, cardiovascular progenitor cells obtained by the method of the present invention, or cardiomyocytes, smooth muscles or endothelial cells derived thereof, including the testing of pharmaceutical compounds for their effect on cardiac, smooth muscles or endothelial tissue maintenance, repair, and function recovery. Screening may be done either because the compound is designed to have a pharmacological effect on the cells, or because a compound designed to have effects elsewhere may have unintended side effects on cells of this type. The screening can be conducted using any of the cardiovascular progenitor cells obtained by the method of the present invention, later-stage cardiomyocytes smooth muscles or endothelial cells precursors or terminally cardiomyocytes, smooth muscles or endothelial cells derived thereof.

Form also part of the present invention, other screening applications of the population of primate, preferably human, cardiovascular progenitor cells obtained by the method of the present invention, or cardiomyocytes, smooth muscles or endothelial cells derived thereof, including the testing of pharmaceutical compounds for their effect in term of toxicity, marker expression, receptor binding, , morphology changes (like hypertrophy...), ATP production, oxydative stress, cell viability or contractile activity and electrophysiology on cardiomyocytes cells. Screening may be also done either because the compound is designed to have a pharmacological effect on the cells, or because a compound designed to have effects elsewhere may have unintended side effects on cells of this type.

Thus, the present invention is directed to a method for testing agents for effect on human cardiac cells comprising the steps of:
a) culturing in suitable conditions cardiovascular progenitor cells, or cardiomyocytes derived therefrom, obtained by the method according to the present invention;
b) measuring the transmembrane action potential of at least one cardiovascular progenitor cells, or cardiomyocytes derived therefrom;
c) exposing said at least cardiovascular progenitor cells, or cardiomyocyte derived therefrom, to the agent; and
d) observing whether the action potential of said at least cardiovascular progenitor cells, or cardiomyocytes derived therefrom, changes after the exposure.

In another aspect, the present invention is directed to a method for testing agents for their effect on the electrical properties of the HERG channel in human cardiac cells comprising the steps of:
a) culturing in suitable conditions cardiovascular progenitor cells, or cardiomyocytes derived therefrom, obtained by the method according to the present invention;
b) inserting an electrode into at least one of said cardiovascular progenitor cells in culture, or cardiomyocytes derived therefrom, preferably terminally differentiated cardiomyocyte therefrom;
c) measuring the duration of the transmembrane action potential of said cardiovascular progenitor cells in culture, or cardiomyocytes derived therefrom;
d) exposing the cardiovascular progenitor cells, or cardiomyocytes derived therefrom, to the agent; and
e) observing whether the action potential duration is changed by the agent, as would be the case if the HERG channel is altered.

In another aspect, the present invention is directed to a method for testing agents for their likelihood of triggering delayed after polarization events in human cardiac cells comprising the steps of:
a) culturing in suitable conditions cardiovascular progenitor cells, or cardiomyocytes derived therefrom, obtained by the method according to the present invention;
b) obtaining a chart of the transmembrane action potential of the cardiovascular progenitor cells, or cardiomyocytes derived therefrom, over time;
c) exposing the cardiovascular progenitor cells, or cardiomyocytes derived therefrom, to the agent; and
d) observing whether a delayed after polarization event is triggered by the agent.

In another aspect, the present invention is directed to a method for testing agents for their likelihood of triggering long QT syndrome in human cardiac cells comprising the steps of:
a) culturing in suitable conditions cardiovascular progenitor cells, or cardiomyocytes derived therefrom, obtained by the method according to the present inventions;
b) obtaining a chart of the transmembrane action potential of a plurality of the cardiovascular progenitor cells, or cardiomyocytes derived therefrom, over time;
c) exposing the cardiovascular progenitor cells, or cardiomyocytes derived therefrom, to the agent; and
d) observing whether a long QT syndrome is triggered by the agent in any of the cardiovascular progenitor cells, or cardiomyocytes derived therefrom.

Preferably, the cardiomyocyte derived thereof is selected from the group consisting of atrial-type, ventricular-type and nodal-type cardiomyocytes.

In a preferred embodiment, the measuring includes impaling a cardiovascular progenitor cells, or cardiomyocyte derived therefrom, obtained by the method of the present invention with an electrode.

In another aspect, the present invention is directed to a method for testing agents for their effect on human cardiovascular progenitor cells, or cardiomyocytes derived therefrom, obtained by a method according to the present invention comprising a step of:
a) measuring a physical characteristic of the contraction of the cells;
b) exposing the cells to the agent; and
c) observing any change in the characteristic of the contraction of the cells.

Preferably, the characteristic of the contraction measured is the physical magnitude of the contraction, more preferably the rate of the contraction.

Standard methods well known by the skilled person can be used for assessing the activity of candidate pharmaceutical compounds (see i.e. In vitro Methods in Pharmaceutical Research, Academic Press, 1997, or U.S. Pat. No. 5,030,015). Said methods generally involves combining the cardiovascular progenitor cells obtained by the method of the present invention, or cardiomyocytes, smooth muscles or endothelial cells derived thereof, with the candidate compound, either alone or in combination with other drugs. The investigator determines any change in the morphology, marker phenotype, or functional activity of the cells that is attributable to the compound (compared with untreated cells or cells treated with an inert compound), and then correlates the effect of the compound with the observed change.

Standard methods also well known by the skilled person can be used for assessing the cytotoxicity of candidate pharmaceutical compounds. Said cytotoxicity can be determined in the first instance by the effect on cell viability, survival, morphology, and the expression of certain markers and receptors. Effects of a drug on chromosomal DNA can be determined by measuring DNA synthesis or repair. [3H]-thymidine or BrdU incorporation, especially at unscheduled times in the cell cycle, or above the level required for cell replication, is consistent with a drug effect. Unwanted effects can also include unusual rates of sister chromatid exchange, determined by metaphase spread (see the above cited reference *In vitro* Methods in Pharmaceutical Research, 1997).

Standard methods also well known by the skilled person can be used for assessing the effect of candidate pharmaceutical compounds on cell function. Said cell function effect can be assessed using any standard methods well known by the skilled person, particularly by using assay allowing to observe phenotype or activity of the cardiovascular progenitors, cardiomyocytes, smooth muscles or endothelial cells derived thereof obtained by the method of the present invention, such as marker expression, receptor binding, contractile activity, morphology changes (like hypertrophy...), ATP production, oxydative stress, cell viability or electrophysiology- either in cell culture or in vivo. Pharmaceutical candidates can also be tested for their effect on contractile activity-such as whether they increase or decrease the extent or frequency of contraction on cardiomyocytes. Where an effect is observed, the concentration of the compound can be titrated to determine the median effective dose (ED50).

In another aspect of the present invention, the cardiovascular progenitor cells, or cardiomyocytes, smooth muscles or endothelial cells derived thereof, obtained by the method of the present invention can be used to prepare a cDNA library relatively uncontaminated with cDNA preferentially expressed in cells from other lineages. For example, cardiomyocytes, smooth muscles or endothelial cells are collected by centrifugation at 1000 rpm for 5 min, and then mRNA is prepared from the pellet by standard techniques (Sambrook et al., Molecular cloning : a laboratory manual, 3rd ed. New York: Cold Spring Harbor Laboratory, 2001). After reverse transcribing into cDNA, the preparation can be subtracted with cDNA from undifferentiated ES or iPS cells, other progenitor cells, or end-stage cells from the cardiomyocytes, smooth muscles or endothelial cells or any other developmental pathway.

In another aspect of the present invention, the cardiovascular progenitor cells, or cardiomyocytes, smooth muscles or endothelial cells derived thereof, obtained by the method of the present invention can be used to prepare antibodies that are specific for markers of cardiomyocytes, smooth muscles or endothelial cells or/and their precursors.

Indeed, by positively selecting using the specific cardiovascular progenitor cells, or cardiomyocytes, smooth muscles or endothelial cells derived thereof, obtained by the method of the present invention, and negatively selecting using cells bearing more broadly distributed antigens (such as embryonic cell progeny or iPS with other phenotypes) or adult-derived cardiomyocytes, smooth muscles or endothelial cells, the desired specificity can be obtained. The antibodies of the invention can be used to identify or rescue heart cells of a desired phenotype from a mixed cell population, for purposes such as co-staining during immunodiagnosis using tissue samples, and isolating precursor cells from terminally differentiated cardiomyocytes and cells of other lineages.

In another aspect of the present invention, the cardiovascular progenitor cells, or cardiomyocytes, smooth muscles or endothelial cells derived thereof, obtained by the method of the present invention can be also used for identifying expression patterns of transcripts and newly synthesized proteins that are characteristic for cardiomyocytes, smooth muscles or endothelial cells, and may assist in directing the differentiation pathway or facilitating interaction between cells. Expression patterns of the differentiated cells are obtained and compared with control cell lines, such as undifferentiated ES or iPS cells, other types of committed precursor cells, or terminally differentiated cells.

The use of microarray in analyzing gene expression is well known by the skilled erson (see i.e. Fritz et al Science 288:316, 2000). An exemplary method can be conducted using a Genetic Microsystems array generator, and an Axon Genepix™ Scanner for comparing mRNA preparations from two cells of interest (using for example Cy3 and Cy5-labeled cDNA).

In another aspect, the present invention is directed to a composition or a culture medium used for culturing cells, preferably primate ES cells or embryonic-like state cells, comprising BMP-2 and matrigel from EHS tumor extract as described and preferred above for the method of the present invention.

The present invention is directed to a kit, preferably for its use in a culture medium, more preferably for culturing primate ES cells or embryonic-like state cells, said kit comprising BMP-2 and as described and preferred above for the method of the present invention, more preferably EHS tumor extract matrigel.

Preferably, the composition, the medium or the kit according to the present invention contains 10 ng/ml BMP-2 (± 5 ng/ml).

Other features and advantages of the invention will be apparent from the following detailed description, and from the claims.

### Example 1: Materials and Methods

Knockout^{™} DMEM (Invitrogen cat n° 10829-018)
Knockout^{™} SR serum replacement (Invitrogen cat n° 10828-028) Test batch before using
Nutristem^{™} XF/FF 500ml (Stemgent cat n° 01-0005, defined, xeno-free, serum-free medium, designed to support the growth of human embryonic stem cells (hESC) and human induced pluripotent stem cells (hiPSC)
Trypsin 0.05 %/0.5 mM EDTA 4Na 1X (Invitrogen cat n°25300-054)
Glutamax 200 mM (Invitrogen cat n° 35050-038)
MEM Non essential amino acids 200 mM (Invitrogen cat n° 11140-035)
Mercaptoethanol (Invitrogen cat n° 31350-010) (Make solution at 10⁻⁷ M in PBS and use for 1 month)
RPMI 1640 medium with glutamax (Invitrogen cat n° 61870-010) (RPMI for Roswell Park Memorial Institute medium)
Dulbecco's Modified Eagle Medium high glucose (DMEM) (Invitrogen cat n° 31965-023)
Dulbecco Phosphate Buffered Saline (D-PBS) Ca- and Mg-free (Invitrogen cat n°14200-067)
B27 50X supplement without Vitamin A (Invitrogen cat n° 12587-010)
Human albumin 200 g/l (BioScience Baxter)
Gelatin 2 % solution from bovine skin cell 100 ml (Sigma cat n° G1393)
Mitomycin C 10 mg (Santa Cruz cat n° sc-3514B)
FGF-2 Human recombinant (Peprotech cat n° 100-18B) (FGF for Fibroblast Growth Factor)
BMP-2 Human recombinant (R&D system) (BMP-2 for Bone Porphogenetic protein- 2) StainAlive™ DyLight™ 488 Mouse anti-SSEA-1 Antibody (Stemgent® cat n° 130-095-668)
Collagenase type IV 1g from Clostridium histolyticum (Invitrogen cat n° 17104-019) SSEA1 microbeads (MACS sorter Miltenyi cat n° 130-094-530)
CD15-FITC (BD Biosciences cat n° 332778)
RNA extraction kit (Mini RNA Isolation II Kit^{™} Zymo Research cat n° R1033) SuperScript^{™} II Reverse Transcriptase (Invitrogen cat n°18064-022)
Oligo(dT)₁₂₋₁₈ Primer (Invitrogen cat n° 18418012)
100 mM dNTP Set (Invitrogen cat n° 10297-018)
Real Time PCR reagent: Light Cycler Fast Start DNA MasterPLUS SYBR Green I (Roche cat n° 3515885)
Nkx2.5 (Santa Cruz cat n° sc-14033)
Isl-1 (Santa Cruz cat n° sc-23590)
TBx5 (Santa Cruz cat n° sc-17866)
Mouse embryonic fibroblasts (MEF) (ATCC cat n° CRL-2991)
Human foreskin fibroblasts (HFF2) (ATCC cat n° CRL-2429)
BD Matrigel™ Basement Membrane Matrix 10 ml (BD Biosciences cat n°356231)

### Equipment:

Sterile cell culture dishes and pipettes
500 ml Filtration unit 0.2 µm (Nalgène cat n° 162-0020)
MACS Cell Separation columns LS (Miltenyi cat n° 130-042-401)
QuadroMACS™ Separator (Miltenyi cat n° 130-090-976)
A real time QPCR thermocycler (Roche LightCycler 1.5)

### Example 2: Example of Preparation of reconstituted basement membrane-derived extracellular composition (named "matrigel") from EHS tumor extract (see US 4,829,000 dated on May 9, 1989, Example 1 and Materials)

Materials - Type IV collagen, laminin and heparan sulfate proteoglycan were prepared from the EHS (Engelbreth Holm-Swarm) tumor (Timpl et al, J. Biol. Chem. 254:9933-9937; 1979; Hassell et al, Proc. Natl. Acad. Sci. U.S.A. 77:4494-4498; 1980; Kleinman, et al, Biochemistry 21:6188-6193; 1982). After washing the tumor tissue in 3.4M NaCl, 0.05 M Tris-HCl, pH 7.4, containing protease inhibitors (Orkin et al, J. Exp. Med. 145:204-220; 1977; Timpl et al, supra), the basement membrane matrix was extracted with 0.5M NaCl in 0.05M Tris-HCl, pH 7.4. Laminin was isolated from the 0.5 M NaCl extract as described by Timpl et al, supra. The residue of tumor tissue from lathyritic animals was extracted with 2.0 M guanidine in 0.05 M Tris-HCl, pH 7.4, followed by an extraction with the same buffer containing 0.005 M dithiothreitol to solubilize the type IV collagen (Kleinman et al, supra). Low density heparan sulfate proteoglycan was purified from 6.0 M urea extracts of the tumor by ion exchange chromatography followed by cesium chloride density centrifugation and molecular sieve column chromatography (Hassell et al, supra). Heparin was obtained from Sigma Chemical Company.

### Preparation of "EHS-matrigel" (matrigel derived from EHS tumor extract)

The procedure is based on about 100 g of tumor and all steps are carried out at 4 °C unless indicated otherwise.
1. Homogenize tumor in 200 ml of 3.4 NaCl buffer comprising 3.4 M NaCl; 0.05 M Tris; 0.004 M EDTA and 0.002 M NEM(n-ethylmaleimide)
   Add H₂O to 2 Liters,
   adjust pH to 7.4.
2. Centrifuge at 10,000 RPM for 15 minutes, discard supernatant.
3. Homogenize tumor residue in 3.4M NaCl buffer.
4. Centrifuge at 10,000 RPM for 15 minutes, discard supernatant.
5. Homogenize tumor residue in 3.4 M NaCl buffer.
6. Centrifuge at 10,000 RPM for 15 minutes, discard supernatant.
7. Homogenize tumor in 100 ml of 2 M urea buffer comprising 2 M urea ; 005 M Tris; 0.15 M NaCl ;
   Add H₂O to 2 Liters and
   Adjust pH to 7.4.
8. Stir overnight at 4 °C.
9. Centrifuge at 14,000 RPM for 20 minutes. Save supernatant.
10. Add 50 ml of 2 M urea buffer to the tumor residue and homogenize.
11. Centrifuge at 14,000 RPM for 20 minutes. Save supernatants.
12. Combine supernatants and dialyze vs Tris-saline comprising 0.05 M Tris; 0.15 M NaCl;
   Add H₂O to 2 Liters
   Adjust pH to 7.4.
   Use a graduated cylinder for 1 liter. Add 900 ml of Tris-saline + 5 ml of chloroform (this is a sterilization step).
13. Dialyze 2 hours - rotate bags at end.
14. Change dialyses to Tris-saline alone.
15. Dialyze one more change with Tris-saline.
16. Last dialysis steps should be against media salts such as DMEM or Dulbecco-Vogt or the like.
17. Inside of bag is sterile. Render the outside of the bag sterile with alcohol, rinse hemostat and scissors in alcohol and empty bags in sterile hood into sterile containers. Aliquot as needed. Collagen IV can be optionally added at this stage to the liquid phase in an amount ranging from about 0.1 to 1 mg/ml depending on the desired consistency or strength of the polymerized gel matrix. The thicker gels have been found to be more durable.
18. Immediately cool. Cryopreserve at -20 °C if storage is desired. For gelation: pour extract into desired containers and warm (about 24 °C-35 °C) for 30-120 minutes for polymerization. For a 35 mm petri dish, use less than 1 ml of the extract. Spread it thin.
19. To use the gel as a cell culture substratum, add about 3 ml of suitable growth medium on top of the polymerized gel obtained from step 18 and inoculate the medium with the dispersions of the cells which are desired to be grown. Of course, the growth medium to be used will depend on the type of the cell which is desired to be grown;

It is understood that this example 2 described above for preparing the basement membrane matrigel extracted from Engelbreth-Holm-Swarm mouse sarcoma is for illustrative purpose only and that various modifications or changes in light thereof will be suggested to persons skilled in the art and are to be included within the spirit and purview of this application and the scope of the present invention.

### Example 3: Culture of human pluripotent cells

### A) Preparation of IPs cells

iPS cells were generated from human newborn foreskin fibroblasts. Foreskin fibroblasts were nucleofected or transducted with 4 plasmids encoding for Oct4, Nanog, Sox2, Lin28 at passage 4 maximum iPS colonies were expanded during several weeks of culture and xxx clones were selected for continued expansion and analyses. Each clone had a human ES cell morphology, normal karyotype and expressed telomerase (hTERT), cell surface markers (SSEA-3, SSEA-4, tumor-related antigen (TRA)-1-60, TRA-1-81, TRA-2-49/6E (alkaline phosphatase)), and genes characteristic of human ES cells (Nanog, Sox2, GDF3, Oct4, REX1). The 4 clones demonstrated multilineage differentiation (Tree germ layers) in embryoid bodies and were able to form teratomas. Finally, the cardiogenic capacity of the iPS clones was evaluated with the appearance of beating embryoid bodies and cardiac differentiation on feeder cells. iPS clones are cultured on MEF using KO^{™}-DMEM medium supplemented with mercaptoethanol, glutamine, non essential amino acids, 14 % KOSR and 10 ng/ml FGF2.

### B) hES cells

hES cells used are 16 cell lines from the Technion Institute (NIH approved). Clones demonstrated multilineage differentiation (Tree germ layers) in embryoid bodies and were able to form teratomas. Finally, the cardiogenic capacity of the hES clones was evaluated with the appearance of beating embryoid bodies and cardiac differentiation on feeder cells.hES clones are cultured on MEF using KO^{™}-DMEM medium supplemented with mercaptoethanol, glutamine, non essential amino acids, 14 % KOSR and 10 ng/ml FGF2.

### C) Culture of human pluripotent cells

Human pluripotent cell lines require feeder cells (Mouse Embryonic Fibroblasts (MEF) for iPS or human foreskin fibroblasts (HFF2) for I6) and FGF-2 to maintain their pluripotency.
1- MEF and FSBT are cultured for 3, 4 passages or 20 passages and treated for 2 hours with 10 and 15 µg/ml mitomycin C respectively; cells are washed twice with PBS, trypsinised for 5 min and plated in gelatin (0.1 %)-coated plates at a density of 40,000 cells/cm²_{.}
2- I6 and iPS are thawed out quickly in warm propagation medium, spin down at 800 rpm for 4 min, and resuspended to be plated on feeder cells cultured overnight with ES cell medium without FGF2. Add FGF2 to the plate.
3- I6 cell lines are cultured on FSBT using Nutristem^{™} medium. For iPS, cells are cultured on MEF using KO^{™}-DMEM medium supplemented with mercaptoethanol, glutamine, non essential amino acids, 14 % KOSR and 10 ng/ml FGF2.
4- Medium is changed every day.
5- Cell colonies are dissociated into small cell clusters every 4-5 days using collagenase type IV (I6 and iPS) (see paragraph "Cell passaging" below). It is preferred to never let the pluripotent cells colonies reaching confluence in the dish. Split them at about 70 % confluence.

### Cell passaging

It is preferred that all media, enzymes, PBS should be warmed up at 37°C. Human ES cells and iPS are very sensitive to change in temperature and should remain as long as possible at 37°C.

I6 and iPS splitting: replace the culture medium by KO^{™}-DMEM with 1 mg/ml collagenase type IV (5 ml is enough for a B10 plate). Incubate the plate at 37 °C for 45 to 60 min. Add 5 ml propagation medium without FGF2 and scrape the cells with a 5 ml pipette. Transfer the cells to a 15 ml falcon tube and spin them down at 800 rpm for 4 min at RT. Aspirate the supernatant and resuspend the cells with propagation medium with FGF2 or Nutristem^{™}. Break down the ES cells colonies by pipetting them forth and back 3 to 5 times until getting a homogenous cell suspension. Plate the cells 1/6.

**Table 1: Propagation medium for iPS cells**

| | |
|---|---|
| KnockOutTM-DMEM | 420 ml |
| Glutamine 200 mM | 5 ml |
| Non essential amino acid (100 mM) | 5 ml |
| MercaptoEthanol 10⁻⁴ M | 500 µl |
| (Use the stock 10-4 M solution for one month only) | |
| Serum replacement | 70 ml (14 %) |
| Medium is filtered on 0.2 µm Nalgene Filter unit | |
| FGF2 | 10 ng/ml |

### Exemple 4: Generation and selection of cardiovascular progenitors

1- Human stem cells and iPS are plated on Matrigel™ (reference BD Sciences Catalog number 356230) coated culture dishes and cultured for 24 hours with a medium allowing feeder free cells condition such as Nutristem™ (Table 4) or Stempro (Invitrogen), ReproFF2 (Reprocell), StemMedia™ Nutristem™ XF/FF (Stemgent), mTeSR^{®}1, TeSR™2 (Stem cells technologies), AF Nutritem^{®} hESC XF (Biological industries). Standard medium allowing feeder free cells condition of culture ara well known from the skilled person
2- Human stem cells and iPS are then treated on FSBT or MEF culture dishes for 96 hrs with 10 ng/ml BMP-2 in RPMI 1640 supplemented with 2 % B27, and 0.5 % human albumin (table 3). It is preferred that the cells do not reach confluency at the end of BMP-2 treatment. It is also preferred that BMP-2 is added as soon as small colonies of ES cells appear.
3- Use StainAlive™ DyLight™ anti-SSEA-1 antibody to stain the SSEA1 positive cells. Cells can thus be sorted out using SSEA1-coated Miltenyl beads (Kit) as follows. FACS is done on cells to check the percentage of SSEA1+ cells with an anti-CD15-FITC.
4- Cells are trypsinized and filtered on a 40 µm mesh nylon filter.
5- Cells are incubated for 30 min at 4° C with gentle occasional agitation with anti-SSEA1 antibody-coated Miltenyi beads (20 µl/10⁷ cells) in D-PBS supplemented with 0.5 % BSA and 2 mM EDTA.
6- Cells are transferred to LS Miltenyi columns set on the magnet.
7- Cells are washed two times with 5 ml D-PBS- BSA/EDTA and allowed to be eluted from the column removed from the magnet using 6 ml of D-PBS/BSA/EDTA. The cells which go through are recycled to the cartridge on the magnet and eluted once more. This procedure is repeated three times. Counting the cells revealed 40-50 % for I6 and 20-25 % of SSEA1 positive cells for iPS.
8- Gene expression profile is performed by RT-real time Q-PCR (see paragraph below "RT-PCR and real time quantitative PCR"). A phenotypic analysis of the CD15-positive cells revealed that they express early mesodermal (Brachyury, Tbx6, Mesp1) and cardiac (Tbx20, Mef2c, GATA4, Isl1, Tbx18, Flk1) markers. Nkx2.5 was absent in the SSEA1 negative while detected in the SSEA1 positive cell population. The SSEA1 positive cell population looses expression of pluripotency gene (Lefty, Nanog, Crypto, Sox2). However, this cell population still over-expresses Oct-4A.

### RT-PCR and real time quantitative PCR

Total RNA is prepared after cell lysis using a kit (Zymo research). After reverse transcription using the reverse transcriptase Superscript II according to the manufacturer instructions, Real time PCR is carried out using a set of gene specific primers (see Table 2). 2-6 ng cDNA is used for real time quantitative PCR, performed with a light cycler 1.5 and the SYBR Green fast start kit (Roche, Germany).

The 12 µl reaction mix contained 1 µl of Master SYBR Green I mix, including Taq DNA polymerase, buffer, deoxynucleoside trisphosphate mix, SYBR Green I dye, 3 mM MgCl2 and 0.5 µM of each primer. 2 µl of 10-fold diluted cDNA is added to the mixture. Data are normalized using RT-PCR of the GAPDH mRNA as an index of human cDNA content after reverse transcription. Amplification includes an initial denaturation at 95 °C for 8 min, and 45 cycles of denaturation at 95 °C for 3 s, annealing at 65°C for 8-10 s, and extension at 72 °C for 7-10 s. The temperature transition rate is 20 °C/s. Fluorescence is measured at the end of each extension step. After amplification, a melting curve is acquired by heating the product at 20° C/s to 95 °C, then cooling it at 20 °C/s to 70 °C. The reaction was maintained at 70 °C for 20 s followed by slow heating at 0.3 °C/s to 95 °C. Melting curves are used to determine the specificity of PCR products, and they are further confirmed by gel electrophoresis.

**Table 2 : PCR primer sequences**

| Genes | Forward (SEQ ID NO:) | Reverse (SEQ ID NO:) |
|---|---|---|
| β-tubulin | CCGGACAGTGTGGCAACCAGATCGG(1) | TGGCCAAAAGGACCTGAGCGAACGG(2) |
| Nkx2.5 | CATTTACCCGGGAGCCTACG (3) | GCTTTCCGTCGCCGCCGTGCGCGTG (4) |
| Mef2c | AGATACCCACAACACACCACGCGCC(5) | ATCCTTCAGAGAGTCGCATGC (6) |
| SRF | CTCCGCCCCGCTCAGACCCCACCACAGA(7) | AGGTAGTTGGTGATGGGGAAGGA (8) |
| α-actin | CTATGTCGCCCTGGATTTTGAGAA (9) | TGAGGGAAGGTGGTTTGGAAGAC (10) |
| Oct-4 | ACGACCATCTGCCGCTTTGAG (11) | GCCTCTCACTCGGTTCTGAT (12) |
| Tbx6 | AGGCCCGCTACTTGTTTCTTCTGG (13) | TGGCTGCATAGTTGGGTGGCTCTC (14) |
| Isl1 | CATCGAGTGTTTCCGCTGTGTAG (15) | GTGGTCTTCTCCGGCTGCTTGTGG (16) |
| FoxH1 | GCCCCTGCCCACGCTGTCTA (17) | GGTACCTCTTCTTCCTCCTCTT (18) |
| Brachyury | CGGAACAATTCTCCAACCTATT (19) | GTACTGGCTGTCCACGATGTCT (20) |
| Mesp1 | CTCGTCTCGTCCCCAGACT (21) | AGCGTGCGCATGCGCAGTT(22) |
| Tbx20 | CTGAGCCACTGATCCCCACCAC (23) | CTCAGGATCCACCCCCGAAAAG (24) |
| Gata4 | GGTTCCCAGGCCTCTTGCAATGCGG (25) | AGTGGCATTGCTGGAGTTACCGCTG(26) |
| Pax6 | GCCAGCAACACACCTAGTCA (27) | TGTGAGGGCTGTGTCTGTTC (28) |
| α-FP | ACTGCAATTGAGAAACCCACTGGAGATG (29) | CGATGCTGGAGTGGGCTTTTTGTGT (30) |
| Cx43 | TACCATGCGACCAGTGGTGCGC(31) | GAATTCTGGTTATCATCGGGGAA (32) |
| GAPDH | ATGGGCAAGGTGAAGGTCGGAG (33) | TCGCCCGACTTGATTTTGCAGG (34) |
| Tbx5 | TACCACCACACCCATCAAC (35) | ACACCAAGACAGGGACAGAC (36) |
| LeftyA | GGGAATTGGGATACCTGGATTC (37) | TAAATATGCACGGGCAAGGCTC (38) |
| TDGF1 | ACAGAACCTGCTGCCTGAAT (39) | ATCACAGCCGGGTAGAAATG (40) |
| Nanog | CAAAGGCAAACAACCCACTT (41) | TCTGCTGGAGGCTGAGGTAT(42) |

**Table 3: Medium for cardiovascular progenitors generation**

| | |
|---|---|
| RPMI 1640 | 478 ml |
| B27 complement | 10 ml |
| Human serum albumin (200 g/l) | 12.5 ml |
| BMP-2(100 µg/ml) | 10 ng/ml |

**Table 4: Feeder cells free media type Nutristem™**

| |
|---|
| - Stempro (Invitrogen, cat n°A1000701), |
| - ReproFF2 (Reprocell, cat n°RCHEMD006), |
| - StemMedia™ Nutristem™ XF/FF (Stemgent, cat n°130-095-543), |
| - mTeSR^{®}1, TeSR™2 (Stem cells technologies, cat n° 05857, 05860 respectively), |
| - AF Nutritem^{®} hESC XF (Biological industries, cat n° 05-100-1A). |

## Claims

1. A method for the *in vitro* preparation of cardiovascular progenitors cells from mammalian ES cells or from mammalian embryonic-like state cells, wherein said method comprises the following step of:
a) culturing of mammalian ES cells or embryonic-like state cells in a medium containing suitable agents allowing their proliferation and maintaining their pluripotency;
b) differentiating the mammalian pluripotent ES cells or embryonic-like state cells obtained in step a) toward cardiovascular progenitor cells by suspending said pluripotent ES cells or embryonic-like state cells in a medium containing BMP-2 (Bone Morphogenetic Protein 2); and, optionally
c) selecting and collecting the differentiated mammalian ES cells or mammalian embryonic-like state cells obtained in step b) which display the SSEA1 marker at their membrane surface, the mammalian ES cells or embryonic-like state cells displaying said SSEA1 marker being selecting and collecting as cardiovascular progenitor cells, wherein in step b) said medium containing BMP-2 further contains a reconstituted basement membrane-derived extracellular composition (named "matrigel").

2. The method according to claim 1, wherein said matrigel in step b) is a reconstituted basement membrane-derived extracellular composition from EHS tumor extract.

3. The method according to claim 1 or 2, wherein said matrigel in step b) is a reconstituted basement membrane-derived extracellular composition containing in parts by weight about 60-85% laminin, 5-30 % collagen IV, 1-10 % nidogen, 1-10 % heparan sulfate proteoglycan and 1-5 % entactin and wherein said composition can polymerize into a rigid stable gel in at least 20 minutes on heating at 24 °C-32 °C.

4. The method according to one of claims 1 to 3, wherein said matrigel in step b) is a reconstituted basement membrane-derived extracellular composition from a tumor, preferably from EHS tumor extract, and obtained by a process carried out at about 4 °C and comprising the steps of:
(a) homogenizing the tumor multiple times in a suitable first buffer containing at least 3.4 M salt and discarding the soluble fraction after each homogenization so as to remove undesirable components;
(b) extracting the residual tumor in a suitable urea buffer containing at least 2M urea keeping the extract stirred for about 16-18 hours;
(c) separating the extract form step (b) and saving the extract while repeating step (b) with the residual tumor fraction and again saving the extract resulting therefrom;
(d) combining the extracts from step (c) and dialyzing against a suitable sterilizing buffer with multiple changes of the dialyzing buffer;
(e) further dialyzing against a suitable buffer not containing a sterilizing component;
(f) recovering a dialysate from step (e); and
(g) polymerizing the dialysate at 24 °C-32 °C for at least 20 minutes.

5. The method according to claim 1, wherein said matrigel is the BD Matrigel™ Basement Membrane Matrix from BD Biosciences, particularly the matrigel contained in the reference BD Biosciences cat n°356231).

6. The method according to one of claims 1 to 6 , wherein mammalian cells are selected from the group consisting of primate cells, mouse cells or rat cell, primate cells being the most preferred.

7. The method according to claims 1 to 6, wherein in step b), the ES cells or the embryonic-like state cells are treated with 10 ng/ml BMP-2 (± 5 ng/ml) and on said matrigel, preferably for respectively 48 hrs and 6 days, more preferably for 96 hours.

8. The method according to claims 1 to 7, wherein in step b), the ES cells or the embryonic-like state cells are for 96 hours with 10 ng/ml BMP-2 in RPMI supplemented with 2 % B27, 0.5 % human albumin and on said EHS tumor extract.

9. A substantially purified population of cardiovascular progenitor cells obtained by the method according to one of claims 1 to 8, wherein the cardiovascular progenitor cells display at their membrane surface the CD15 marker and, preferably retain their capability to proliferate and repopulate the postinfarction scar when they are administrated to a patient in need thereof.

10. The substantially purified population of cardiovascular progenitor cells according to claim 9, wherein said cardiovascular progenitor cells further express the the early mesoderm Brachyury, Mesp-1 and Tbx6 markers, the cardiovascular Tbx20 and Mef2c markers, the GATA4, Nkx2.5, Isl1, Tbx5, Tbx18, MyoCD and flk-1markers and the Oct-4A marker.

11. Composition comprising a substantially purified population of cardiovascular progenitor cells according to claim 9 or 10 for its use as a medicament.

12. Composition comprising a substantially purified population of cardiovascular progenitor cells according to claims 9 or 10, for treating heart failure in a primate, preferably in a human.

13. The substantially purified population of cardiovascular progenitor cells or cardiomyocytes, endothelial and smooth muscles cells derived therefrom, according to claims 9 or 10 for its use in a screening method for identifying whether an agent of interest has potential or no potential side effects or direct efficacy effects on cardiomyocytes, endothelial or smooth muscles cells, particularly toxicity side effect.

14. Method for testing agents for effect on human cardiac cells comprising the steps of:
a) culturing cardiovascular progenitor cells, or cardiomyocytes derived therefrom, according to claim 9 or 10;
b) measuring the transmembrane action potential of at least one cardiac progenitor cell, or cardiomyocyte derived therefrom;
c) exposing said at least cardiovascular progenitor cell, or cardiomyocyte derived therefrom, to the agent; and
d) observing whether the action potential of said at least cardiac progenitor cell, or cardiomyocyte derived therefrom, changes after the exposure.

15. A composition or a culture medium or a kit used for in vitro culturing cells, preferably mammalian ES cells or mammalian embryonic-like state cells, more preferably primate cells, particularly human cells, said composition or culture medium or kit comprising BMP-2, or functional fragment thereof, and a composition of EHS tumor extract as defined in one of claims 1 to 5.
